(19) Europäisches Patentamt
European Patent Office
Office européen des brevets

(11) EP 1 363 951 B1

(12) EUROPEAN PATENT SPECIFICATION

(45) Date of publication and mention of the grant of the patent:
**25.10.2006 Bulletin 2006/43**

(21) Application number: **01999583.6**

(22) Date of filing: **07.12.2001**

(51) Int Cl.:
*C08B 37/00* (2006.01) *C12P 19/10* (2006.01)
*A61K 33/26* (2006.01) *A61K 31/715* (2006.01)

(86) International application number:
**PCT/YU2001/000031**

(87) International publication number:
**WO 2002/046241 (13.06.2002 Gazette 2002/24)**

(54) **POLYNUCLEAR COMPLEX FE(III) WITH PULLULAN OLIGOMERS, PROCESS OF ITS OBTAINING, AND PHARMACEUTICAL PREPARATIONS ON THE BASIS OF THE COMPLEX**

MEHRKERNIGER KOMPLEX VON FE(III) MIT PULLULAN-OLIGOMEREN; VERFAHREN ZU DEREN HERSTELLUNG UND PHARMAZEUTISCHE ZUSAMMENSETZUNGEN AUF BASIS DIESES KOMPLEXES

COMPLEXE POLYNUCLEAIRE FE(III) CONTENANT DES OLIGOMERES DE PULLULANE, METHODE DE PRODUCTION, ET PREPARATIONS PHARMACEUTIQUES OBTENUES A PARTIR DU COMPLEXE

(84) Designated Contracting States:
**CH DE DK LI**
Designated Extension States:
**MK**

(30) Priority: **07.12.2000 YU 76900**

(43) Date of publication of application:
**26.11.2003 Bulletin 2003/48**

(73) Proprietor: **AD "Zdravlje" Farmaceutsko - Hemijska Industrija,
Centar Za Istrzivanje I Razvoj
16000 Leskovac (YU)**

(72) Inventors:
- **ILIC, Ljubomir
YU-16000 Leskovac (YU)**
- **RISTIC, Suzana
YU-16000 Leskovac (YU)**
- **CAKIC, Milorad
YU-16000 Leskovac (YU)**
- **NIKOLIC, Goran
YU-16000 Leskovac (YU)**
- **STANKOVIC, slobodan
YU-16000 Leskovac (YU)**

(74) Representative: **Merdzhanov, Mihail Zahariev
6, Triaditsa Str.
Office 402
1000 Sofia (BG)**

(56) References cited:
**WO-A-00/61191** **GB-A- 1 111 929**

- **CHEMICAL ABSTRACTS, vol. 134, no. 26, 25 June 2001 (2001-06-25) Columbus, Ohio, US; abstract no. 373471e, CAKIC M. ET AL.: "FTIR study of hydrates and their partial deuterated analogs of pullulan and its complex with iron(III)." page 1326; XP002197871 & PHYS. CHEM 2000, PROC. INT. CONF. FUNDAM. APPL. ASPECTS PHYS. CHEM., 5TH 2000, pages 640-642,**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

### *The field of technique*

The suggested invention belongs to the field of polymeric products and relates to the novel product iron(III) polynuclear complex with pullulan oligomers, and process for its obtaining, as well as to the pharmaceutical preparations based on the complex. Pullulan, amorphous, in water soluble polymer, is described as a neutral polysaccharide with linear inflected chain, whose main axis consists of the units α-D-glucose). Structural unit, which is regularly repeated in pullulan, is maltotriose (α-1,4-triglucoside) bound by α-1,6-glycoside bonds as shown by structural formula (I), and therefore, pullulan is more suitable for obtaining of the preparations based on iron(III)-complex, primarily reflected in non-toxicity and absence of side effects.

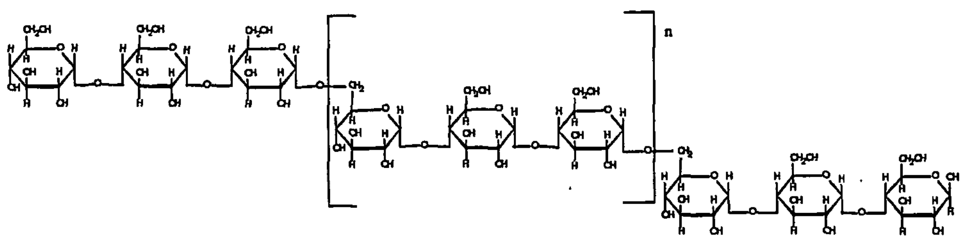

(I)

This complex, which is by means of subsequent standardization modified to the preparation, is applied for prevention and treatment of syderopenic anemia in human and veterinary medicine. The preparation applied intravenously (i.v.) and intramuscularly (i.m.) is characterized with complete non-toxicity, in contrast to the complexes already existing at the market.

The use of native pullulan (n=100-8.200, n-level of polymerization of maltotriose) at wide range of molar mass ($M_w$ =50.000-4.000.000 $gmol^{-1}$) as ligands for sythesis of iron complex is mentioned by other authors in brief announcements (G. Nikolic, M. Cakic, Lj. Ilic, C.A. vol. 134, No. 26, Int.Conf.Pyhs.Chem.2000, Beograd), considering hydrates and deuterationed analogs of pululan with the method of IR spectroscopy. For the purpose of indentification of crystalline form of some segments of the pullulane macro molecules, the number and type of the water molecules in the structure of polysaccharides, the authors are noticing the possibility and way of making complexed pullulan analog to dextrin, but they are not analysing the process of synthesis. The accent is put on the development of new spectroscopic method for establishing of the nature of the connections of polysaccharides and water molecules with various metals.

### *Technical problem*

Subject of the invention is the new product iron(III) polynuclear complex with pullulan oligomers as well as the process for obtaining of this complex, suitable for preparation of injection solution intended mainly for prevention and therapy of syderopenic anemia in humans and animals, characterized with good iron absorption, non-toxicity and acceptable economic and technological criteria.

Regarding the preparation for intravenous administration, it is particularly important and high demands are set, concerning stability of the solution and bioavailability of iron, which at the same time guarantee absence of precipitation *in vivo* due to the influence of tissue liquids, proteins, and electrolytes. This invention attains such an effect, and at the same time, it also attains high therapeutic efficiency (synthesis of hemoglobin), low toxicity, absence of anticoagulative, chemolytic, alergic, and antipreventive effects, which give the preparation an advantage over antianemics, already existing at the market.

### *Background of the invention*

One of the important microelements, necessary for growth, development and maintenance of vital functions in organism, is iron. Iron is necessary for synthesis of hemoglobin and has a positive influence on erythrocyte and reticulocyte number and hematocrite value. Iron insufficiency in organism, as a consequence has occurrence of sideropenic anemia, the illness occurring very frequently in our country and world wide at any age. Treatment of iron insufficiency is very successful when proper amount of iron are orally given in forms of salt, through different medicines (ferrous sulphate, ammonium ferrous sulphate, ferrous succinate, ferrous fumarate, ferric-sorbitol-citrate complex, ferric sulphate, ferrous succinate, ferrous fumarate, ferric-sorbitol-citrate complex, ferric ammonium oxalate, etc) where therapeutic effect depends on free iron concentration. In practical use, nowadays, a great number of preparations, with considerable mutual differences concerning their chemical, physical and pharmacological characteristic, is present on the market. According to the traditional classification, based on chemical properties only (A. Muller, Arzneim.Forsch., 24(6), 880-883(1974)), all antianemic remedies are classified in four basic groups: iron salts, low-molecular iron chelates, low-molecular sandwich-complexes and polynuclear ferric hydroxide complexes with carbohydrates.

In treatment of sideropenic anemia, parenteral preparations on the basis of dextran complex and its derivatives with iron appeared to be the most suitable ones. Good therapeutic properties of aqueous colloidal suspensions of these compounds are not accidental because these iron complexes are similar to the physiologically active protein with iron, Ferritin.

The task of forming the new, physiologically active, iron(III)-complexes, will be as relevant as iron deficient anemia is widely spread in the world, particularly in children and women. Concerning the fact that it is, even nowadays, present in 30% of the world population, even in spite of the extraordinary achievements of biology, pharmacology and medicine sciences, sideropenic anemia represents a significant problem both in human and veterinary medicine.

In patent and non-patent literature a great number of procedures for obtaining of all stated forms of the preparations on the basis of iron, is described. They differ among themselves concerning kind and quality of starting raw-materials, as well as concerning the course and regime of the process of synthesis and standardization of the complex.

Iron-dextran complexes are well known in human medicine, as well as in veterinary practice, for 40 years. Ligands suitable for complexation, are obtained by hydrolytic depolymerization of crude dextran with high average molar mass ($M_w$). Removing of the unwanted fraction of mono-, di- and lower oligosaccharides ($M_w$<1000 g/mol) from the system, because it was determined that they decompose during synthesis to the form of the toxic products (D.Schwengers, U.S.Pat.No.4,927,756), gave products which can be converted into some derivatives such as: hydrogenated dextran (N.A.Floramo, U.S.Pat.No. 3,234,209), oxidized dextran (dextran-carboxylic acid) (B. Pekiċ, D. Cvetkoviċ, YU.Pat.No. 42,384 B), carboxymethyl-dextran (L.I.Novak, U.S.Pat.No. 2,856,398), dextran-heptonic acid (U.S.Pat.No. 3,536,696 and 3,639,588), with subsequent synthesis , under different conditions of temperature and pH, to obtaining of the suitable complexes with iron.

Polynuclear iron(III)-complex solutions can also be obtained with other ligands of polysaccharide type. Complex of dextrin with iron is known. In contrast to dextran, the complex with dextrin is not cumulative due to metabolizing enzymes present in the body, and it does not form harmful antibodies. However, the bad characteristic of the complex is that it is unstable during storage.

The latest inventors improved some of the characteristics of the preparations based on dextran, in the sense of reaction of oleate compound of reactive polynuclear iron(III)-complex (U.S.Pat.No. 4,189,474), however, high i.v.toxicity ($LD_{50}$=460 mg/kg) still persisted.

Preparations obtained with polycarboxylic acids of dextran and colloidal ferric hydroxyde (U.S.Pat.No. 4,370,476), showed good stability and high iron content with very small portion of free iron (<0,05%), but acute toxicity is high ($LD_{50}$=500 mg/kg).

Concerning pullulan, as a ligand it was mostly used for obtaining of pullulan sulphates and the salts thereof (France Pat.No. 2,287,911). Patents belonging to Japanese authors protect the process for obtaining of cyanoethylated pullulan ether (Japanese Pat. 59,226,001A) and obtaining of pullulan polyelectrolytic complex (Japanese Pat. 60,156,702A).

In the survey of patent literature (Jap. Pat. 5,424,419) pullulan is mentioned as one of possible ligands in obtaining of iron complexes, but the authors do not take up these problems. Their invetion relates, first of all, to obtaining of oxidized form of dextran and conditions for convertion into complexes with ferrous and ferric iron forms, not discussing the problems of hydrated form of dextran and its capability of complexing.

Obtaining of crude pullulan, which is obtained as a viscous substance, by centrifuging separated from the biomass and purified by precipitation with ethanol, is described in the paper of S. Liu. A. Steinbüchel., Appl. Microbiol Biotechnol (1996); 46:273-278).

***Description of technical problem solution with examples***

This invention relates to the novel complex obtained in the process of synthesis of polysaccharide the oligomers of pullulan with iron(III)-hydroxyde. The invention also provides the method for obtaining of iron-pullulan preparation as a new antianemic. Obtained preparations comprise aqueous colloidal suspensions or the complex iron(III)-hydroxide solutions with pullulan oligosaccharides with average molar mass in range of 2000-20000 g/mol primarily 7000-12000 g/mol. Pullulan complex with iron(III)-hydroxyde is an aqueous dark red solution, stable at temperatures in range 4-50˚C, during period of 5 years, stable concerning air oxygen and light, with time of complex hydrolytic decomposition in range 5 to 7 seconds. Novelity of the invention is also that some pullulan derivatives, such as hydrogenated oligomers and oxidized oligomers, can form stable colloidal complexes with iron(III)-hydroxid. It was found that stated ligands, with low intrinsic viscosity (mostly in range of 0,02-0,08 dl/g), produce important injection solutions. The resulting complex with

iron content in range of 50-75 $mgFe^{3+}$/ml in the preparation, is not toxic, but it possesses high degree of iron resorption from the location of i.m. application (13,5 $mgFe^{3+}$/100ml of serum after 24 hours).

Structure examinations of this complex mostly showed that it concerns globular system composed of FeOOH globular particles (polymorph forms), wrapped with polymer molecules of the ligand, partially bound by hydrogen bonds through the hydroxyl groups with FeOOH, and partially by coordinate covalent bond through free electron pairs of oxygen.

***Structure of the complex***

*UV- VIS spectrophotometry*

UV - VIS spectra of iron(III)-complex with pullulan are almost identical with those of the complex with dextran (Figure 1). The bands with the maximal absorptions at $\lambda$ = 207 and 308 nm are characteristic for carbonyl chromophors of the ligand investigated. Certain bathochromic shifts on $n \rightarrow \pi^*$ transition od depolymerized pullulan (at aproximately 300 nm), compared to glucose, can be explained by the change of polymerization degree. Extension of the polymer chain increase the energy level of n-electron in basic state, while the $\pi$ level is not significantly changed. For that reason, less energy is needed for $n \rightarrow \pi^*$ transition, and therefore, the maximum is bathochromicly shifted.

Diluted solution of the complex (20 $\mu$l in 100 ml of water) also shows a very weak absorption in VIS area EMZ. Absorption peak appears at approximately 500 nm, as a consequence of the $d \rightarrow d$ transition in iron, which requires the energy of visible light. It is also confirmed by the value of molar energy content of radiation, which is $E_m$ = 239,4 $kJ.mol^{-1}$. But, since the $d \rightarrow d$ transition is forbiden according to the Laporte's rool, appearance of the band is explained by overlapping of the d-orbits of the central metal ion with the p-orbits of the ligand, so that the the $d \rightarrow d$ transition is actually the $p \rightarrow d$ transition, which is allowed. Taking in consideration that interference of the corresponding d- and p-orbits is extremely weak, so the intensity of the corresponding band is low. This fact, similarly to the other spectroscopic methods, points to the existance of a very weak bond between iron and the ligand, probably, just hydrogen bond by available xydroxyl groups.

Characterization of the complex, using the methods of UV-VIS spectrophotometry, confirms the presence of iron in octahedral surrounding of the ligand (calculated value of energy, equal to 2.57 eV, is characteristic for tearing in octahedral field).

*FTIR spectrophotometry*

FTIR spectrum of FeOOH, obtained by alkalous precipitation from ferric chloride solution, is shown on Figure 2. Presence of a wide, intensive band in the spectrum at 3400 $cm^{-1}$ as well as the one at approximately 690 $cm^{-1}$, according to frequency, shape and intensity, should be attributed to $\nu_{OH}$ and $\delta_{OH}$ vibrations of the OH groups from the bond H-O···Fe, respectively. Frequencies of mentioned vibrations point to $\beta_2$ - modification of FeOOH.

The band at approximately 1625 $cm^{-1}$, the result of the $\delta_{OH}$ vibration, points to the presence of water of crystallisation in the structure of FeOOH. Using the method of FTIR spectroscopy, it was determined that $\beta_2$ - modification of FeOOH, in addition to the water of crystallisation, also contains in its structure around 4% of adsorbed water. The molecules of water involved in formation of hydrogen bond with the OH groups from FeOOH. Superficial Fe atoms are octahedrally coordinated by $H_2O$ ligands, forming the first hydrated layer. These are bound to the molecules of water from the second hydrated layer, by the weak hydrogen bond.

The bands with medium intensity at approximately 430 $cm^{-1}$ are attributed to the stretching Fe-O···Fe vibrations. Within the same spectrum, there are bands at 1480 $cm^{-1}$ and at around 1350 $cm^{-1}$, which according to their shape, intensity and frequency, should be attributed to bending OH vibrations of FeOOH in the plane.

In the spectrum of pullulan oligomers (Figure 3), as the most characteristic bands in the area 3700 - 3000 $cm^{-1}$, 2980 - 2800 $cm^{-1}$, the band at around 1640 $cm^{-1}$, then in the area 1500 - 1300$cm^{-1}$, 1200 -1000 $cm^{-1}$ and 950 - 400 $cm^{-1}$ can be observed.

The intensive wide band at approximately 3400 $cm^{-1}$ is conditioned by the stretching vibrations of the OH groups involved in formation of hydrogen bonds. The less intensive bands in the area 2980 - 2800 $cm^{-1}$ originate from the stretching vibrations of the CH and $CH_2$ groups. A band at 1640 $cm^{-1}$ originates from the deformative OH vibrations belonging to the molecules of water. Relatively clear, but complex band in the area 1500 - 1300 $cm^{-1}$ is related to the bending vibrations in the plane of the $CH_2$ groups. Strong bands in the area 1200 - 1000 $cm^{-1}$ are conditioned by stretching vibrations of C-O groups, belonging both to skeleton as well as to glycoside. The bands lower than 900 $cm^{-1}$ originate from the bending vibrations of the methyl groups out of the plane, CH bonds and the pyran ring itself. In the area 700 - 400 $cm^{-1}$ bending vibrations out of the plane from the OH groups are expressive. Existence of $\alpha$-1,4-glucoside bonds of the maltotriose unit of pullulan is characterized by the bands at 756 and 930 $cm^{-1}$, while the bands with weaker intensity at 772 and 915 show that these units are mutually bound by $\alpha$-1,6-glucoside bonds.

FTIR spectra of native, hydrolysed pullulan and its hydrogenated oligomers, are basicly very similar. The

resemblance of the spectra, particularly in the bending area, points to the fact that during the process of hydrolyse and hydrogenization of obtained oligomers, change of glucopyranose units conformation does not take place. In this area (Figure 3a), at least two bands with weaker intensity were registered, at 930 and 850 $cm^{-1}$, they can be assigned as C-CH bending vibrations from the ring, and are characteristic for C1 chair conformation of glucopyranose units.

Mutual comparison of the IR spectra of the complex (Figure 4) and integral components (Figures 2 and 3), in the area of $\nu_{OH}$ vibrations, has shown a movement of the cenrtoid of the band toward lower frequencies. It points out that in the complex, stronger hydrogen bonds are formed than in corresponding starting compounds (probably between the OH groups of FeOOH and oligosaccharides). During establishing of hydrogen bonds, essential role plays $CH_2$-OH group, whose space arrangement changes probably due to the possibility of free rotation around the C-C bond. Analysis of IR spectra in low-frequency area (lower than 700 $cm^{-1}$), did not show presence of any new, nither changes of already existing bands from the valence Fe-O vibrations. Namely, in this area, the band from $\nu_{FeO}$ vibration from FeOOH already exists (460 $cm^{-1}$), so, the effect of accidental degeneration of the two different $\nu_{MO}$ vibrations cannot be excluded, which is hardly probable taking into consideration the appearance of the spectra in all area of the C-O vibrations of the ligand.

Analysis of the spectra in area 1000 - 700 $cm^{-1}$ shows that the complex with pullulan oligomers maintains the same appearance in the $\gamma_{CH}$ area. Similarity of the C-CH bending modes shows that the C1 chair conformation of the glucopyranose unit is represented here too, the units are mutually bound by $\alpha$-1,4 and $\alpha$-1,6 bonds.

Presence of the specific form of iron in the complex, whose band is expected at about 700 $cm^{-1}$ (the position maked with * on the Figure 4), cannot be precisely determined due to the considerable overlapping of the bands belonging to $\gamma_{OH}$ hydrogenated pullulan and FeOOH itself. However, presence of the band of the Fe-O bond at the same frequency (at 470 $cm^{-1}$) shows that in the comlex the $\beta_2$-form of the FeOOH component is probablly maintained.

Comparing the $\delta_{OH}$ areas of the IR spectra (the band at around 1630 $cm^{-1}$), protonated and partially deuterated (using isotopic exchange method) complex analogues and ligands, it is determined that all the compounds are crystallohydrates, containing one crystallographic type of water molecule in their structure. Water protons are involved in fomation of different (concerning strength) hydrogen bonds (estimated $O_w$ ....O distance, on the basis of Berglund correlation, amount 284 pm for pullulan and 290 pm for the complex).

*Scanning electronic microscopy*

Scanning electronic microscopy (SEM) represents relatively good basis in structure analysis of complex compounds. In analysis of polynuclear iron(III)-complex with pullulan oligomers, in order to define its morphologic characteristics, two methods were used: transmission (JEM 100 CX Jeol) and scanning (JSM 5300 Jeol) electronic microscopy.

On the shown TEM micrographs (Figure 5), it can be observed that both FeOOH and the complex contain particles with approximately spherical shape. The first characteristic which can be distinguished from the micrograph, is existance of different particles' sizes, with the most dominant one with about 8 nm in diameter. Another characteristic points to a very uniform percentage of the particles with similar dimensions, their uniform distribution of their sizes is noticeable in range of 5 - 20 nm in diameter. Small spherical colloidal particles do not show any tendency to form larger aggregates. The results of morphologic analysis are shown in Table 1.

Table 1. Morphologic analysis of particle FeOOH diameter and polynuclear iron-pullulan complex distribution

| Description | $\beta_2$-FeOOH (nm) | Complex (nm) | $\Delta$ (nm) |
|---|---|---|---|
| total amount of the objects observed | -3520- | -3542- | - |
| average value | 8 | 12 | 4 |
| minimum | 3 | 6 | 3 |
| maximum | 18 | 25 | 7 |
| mediana | 10 | 15 | 5 |
| size range | 15 | 19 | 4 |
| standard deviation | 21.3 | 32.4 | - |

The obtained results (Table 1) points to presence of the colloidal particles in the complex. Comparison of dimensions of examined particles with those of similar systems shows that proportions of iron-pullulan particles are typical, while the increase of 3-7 nm from FeOOH to the complex, is on the other hand very similar. According to the literature data, it originates from the electron-transparent layer of the ligand surrounding the electron-impermeable core of the FeOOH particle.

Satisfactory iron-polysaccharide microspheres can be formed with pullulan with molar mass in range of 7.000

- 15.000 g-mol[-1]. While the complexation is carried out by neutralization of ferric chloride solution in presence of poly- and oligosaccharide in excess, it is reasonable to assume that the colloidal FeOOH particles get wrapped with pullulan, as a stabilizing hydrophilic covering, in, for the time being, unknown manner. Thus, some of the complex particles acquire optimal size for vizualization of the surface also by means of scanning electronic microscopy. The corresponding SEM micrographs are shown on Figure 6.

SEM micrographs show spherical particles, the size of each up to approximatelly 0,1 μm in diameter. However, with this vizualization, one gets an impression that single particles wrapped with a specific layer, probably oligosaccharides, get united. In accordance with that, considerable differences in sizes of particles are also more understandable.

***Synthesis of complex***

**Synthesis of complex** is carried out when a solution of hydrogenated or oxidated pullulan oligomer reacts with synthetized FeOOH, according to given concentration ratio. The mixture is roughly homogenized with stirring, by mild heating in range 30 to 60˚C, primarily at 50˚C, and then, 0,5M NaOH solution is used to adjust pH of synthesis in range 7 to 12. Roughly homogenized reaction mixture is further heated in closed system, under pressure in range $1{,}013x10^5$Pa to $4{,}5x10^5$Pa at synthesis temperature in range 80 to 250˚C, primarily in range 100 to 160˚C, for 1 to 12 hours. Complete dissolution of ferric(III)hydroxide and obtaining of clear reaction mixture, confirm the successful synthesis of complex. When the synthesis is completed, pH of obtained complex is adjusted to 5,5 to 7,5, and deionization of the solution is carried out on acid and base ion exchangers (Amberlit IRA - 120 and Amberlit IRA - 410), complex pH is adjusted again, filtration through Seitz filter is carried out, and the solution is evaporated in vacuum evaporator to the certain volume.

This synthesis converts an inorganic, insoluble macromolecule of iron(in)-hydroxyde, into a water soluble organic-inorganic complex, by linking to hydroxyl groups of pullulan - main ligand in this complex, which can be successfully incorporated into pharmaceutical preparations for prevention and therapy of sideropenic anemia. This invention, through a simple synthesis, solves the problem of obtaining of the complex which is stable, non-toxic, and with low production cost. The complex is later simply incorporated in pharmaceutical preparations for treatment of anemia.

Based on many physico-chemical, chemical-preparative, and pharmaco-biological examinations, existence of many factors, necessary for a successful synthesis of complex, was irrefutablly established. For obtaining of the complex, the synthesis temperature is in range from slightly raised (30-60˚C) to extremely high temperatures (250˚C, but under corresponding pressure). The regime of heating develops during two phases. The first, the shorter one is carried out at lower temperature (up to 60˚C), in order to attain better homogenization within the system. The second phase reaches the temperature necessary for synthesis. As an agent for activation of reaction of complexation, a conviniant mineral base (KOH, $Ca(OH)_2$, most frequently NaOH) must be present in the system, being dosed in accordance with quantity of iron present in raction mixture (up to 15%). Taking into consideration the fact that we have two-phase system, intensive stirring during synthesis must be provided. Duration of reaction is also in wide range from 1 to 12 hours, most frequently 3 to 6 hours, and it is determined beforehand, as the time needed for dissolution of the solid phase of FeOOH in the aqueous phase of the used ligand solution.

Based on observations conducted so far, presence of coloidal iron dispersed in solution of unbound ligand, in addition to iron bound by covalent or hydrogen bond, was determined in all complexes, therefore it is clear that their relationship graetly influences stability of such solutions. For the time being, there is no objective possibility for quantification of these two values, which can be explained by the fact that the same preparations from different producers mutually differs concerning their physical and chemical characteristics. For that reason conditions for synthesis should be adjusted by increasing the share of coordinative bonds between a ligand and iron.

Numerous examinations of polynuclear complexes showed that for obtaining of stable complexes, where complex of iron with carbohydrates belongs, it is necessary to use ligands which do not have reductive properties; they reach relatively low molar mass and homogenous distribution of ligand molar mass.

The state of teminal unit on polysaccharide ligand is very important. In carbohydrates with a free aldehyde or ketone group at the end of the chain (since the synthesis is carried out in alkaline medium), that group takes on the carboxylic function during reaction, which is used to establish the main link with FeOOH. But, due to the fact that carbohydrates are unstable in alkaline medium, and due to their reductioning activity, during synthesis, the ligand is degraded, and iron is reduced, and finally, a complex mixture, containing all of three possible valent states of iron ($Fe^{3+}$, $Fe^{2+}$, $Fe^0$) and ligand degradation products is obtained, with low amount of genuine complex. Therefore, ligands which do not possess reductive activity are more suitable, or the ones with a possibility to convert terminal function into more active carboxylic function, by previous treatment. Reductive activity of a ligand may be excluded by certain procedures of derivation. Most frequently used is derivation achieved by oxidation or reduction of appropriate functional groups. The fact that reductive capability of a ligand plays a significant role is shown in information that in its presence, possibility of aplication of more drastic conditions of synthesis is reduced (increased temperature and higher concetration of alkali).

Pullulan, amorphous, in water soluble polymer, is described as a neutral polysaccharide with linear inflected

chain, whose main axis consists of the units α-D-glucose). Structural unit, which is regularly repeated in pullulan, is maltotriose (α-1,4-triglucoside) bound by α-1,6-glycoside bonds as shown by structural formula (I),

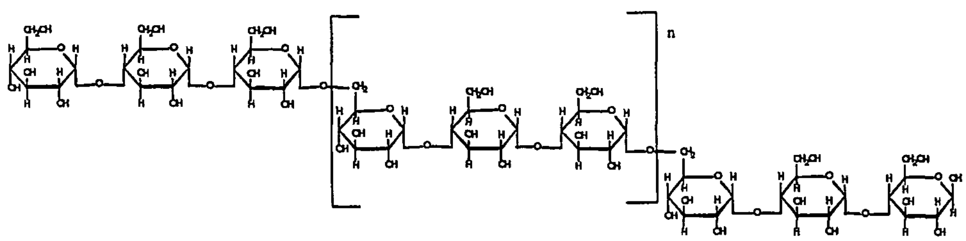

(I)

wherein n=100-8200

and therefore, pullulan is more suitable for obtaining of the preparations based on iron(III)-complex, primarily reflected in non-toxicity and absence of side effects. It is obtained by fermentation of the nutritive medium with substrate (glucose, saccharose, maltose, dextranes, etc) using the fungus *Aureobasidium pullulan.* The process of fermentation lasts for 5-7 days at temperature in range of 25-28,5˚C, with stirring, at pH in range of 6-6,5. Crude pullulan, obtained as a viscous substance, further is by centrifugation separated from the biomass, purified by precipitation with ethanol. It is well soluble in water, and represents a good ligand, whose fractions, with molar masses in range of 1000-25000, are well complexated with iron(III). In order to obtain the polynuclear complex with pullulan, iron(II) salts, as well as magnesium, calcium, manganese, strontium, nickel, cobaltic, cupric, zinc, barium and other similar salts, can be used.

Experimental examinations also pointed out molar mass of the ligand, as an important factor for obtaining of a stable complex. It was established that low- molar mass carbohydrates (mono- and disaccharides) effectively disperse particles of FeOOH, but giving rather unstable sols. The facts point out the existence of a threshold value for molecular weights of carbohydrates, which provides successful linking with iron. For successful synthesis of the complex, as a starting ligand oligomers of native pullulan (white, amorphous, very hygroscopic, tasteless and odourless powder, easily soluble in water, and hardly soluble in methanol, ethanol, propanol, and acetone, with $M_w$ in range 1000 to 25000, and [η] in range 0,01dl/g to 0,16dl/g), obtained by hydrolytic decomposition, were used. The hydrolysis itself can be accomplished with acids, bases, and enzymes. Acid hydrolysis with hydrochloric acid was primarilly carried out. The temperature of depolymerization was in range 60 to 95˚C, and hydrolisis time was in range 30 to 280 minutes. Obtained depolymerizates have an average molar mass in range 1000 to 25000, most frequently 3000 to 12000, and for that reason, they give complexes with high content of iron.

**Depolymerizate of pullulan** may be further modified, for example, by partial reduction with catalysts ($LiAlH_4$, most frequently with $NaBH_4$) in the process of catalytic hydrogenation or oxidation treating with oxidative agent (for example bromine, sodium hypobromite, sodium bromite, sodium hypochlorite, sodium chlorite, sodium periodate, pure oxigen or in combination with inert gases).

For synthesis of the complex of pullulan oligomers with Fe(III), iron(III)hydroxide $\beta_2$-**polymorphous modification** (formula II),

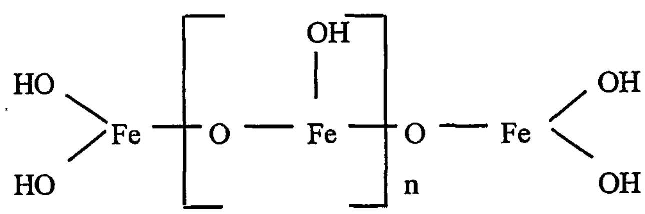

wherein n = 900

(II)

was used, which is, compared to α, γ, and δ polymorphous modifications, the only one obtained by synthesis. It is characteristic that β-modification of iron(III)-hydroxyde appears in two forms: $\beta_1$-FeOOH which combined with carbohydrates does not form any complexes soluble in water, and $\beta_2$-FeOOH in form of gel which shows good capability of formation of complexes with polysaccharides and which was used for synthesis of the complex..

**Ferric hydroxide gel ($\beta_2$ - FeOOH)** is obtained by process of neutralization of iron(III) salts solution with alkali.

Concerning salts, a chloride, nitrate, sulphate, perchlorate, acetate, trichloracetate, as well as binary salts such as iron amonium sulphate or iron amonium citrate my be used. Among them, iron chloride is more suitable than others. Concerning alkali, sodium, lithium, and potassium hydroxides and carbonates are used. Iron(III) gel is best prepared by addition of 0,14-1,24 parts by mass of aqueous $Na_2CO_3$ slowly, dropwise, to the aqueous solution containing one part by mass of hydrated iron chloride.

Before approaching the synthesis, cooled and clear solution of depolymerized pullulan is passed through the column with cation exchanger with flow rate 0,073-0,075 $cm^3/g$ in minute, and remanent quantity of pullulan is extruded with distilled water. pH of such a solution is between 3,6 and 4,4, and it is passed through the column with anion exchanger under the same conditions, while a completely deionized solution (a negative reaction to $Cl^-$ ions with $AgNO_3$) with pH 9,6 to 10 is obtained. Adjust pH to 6,3-6,6 using diluted HCl solution, and excess of water from the sample is removed by evaporation in vacuum to the certain volume. After deionization, solution may be used directly in synthesis of the complex with iron. The same process is applied for ion-exchange chromatography of synthetized complex.

In order to obtain preparation for prevention and treatment of anemia in human and veterinary use it is necessary to perform the subsequent processing of the existing complex. First of all, determine the contents of iron, pullulan, and chloride, adjust the content of chloride to the isotonic solution with NaCl, and then, carry out thermal sterilization in an autoclave for 20 minutes at 120˚C, and under pressure of $1,013x10^5$Pa, to remove possibly present bacteria. After physical and chemical sandardization, the preparation is packed in ampullas.

In order to make understanding of the process, which is object of the invention, easier, examples of different phases of the process, and should not be considered as restrictive.

**EXAMPLE 1: Preparation of crude pullulan**

Crude (native) pullulan is obtained with strain *Aureobasidium pullulans* CH-2 in fermentor on the medium with following content: 10% of one of the known substrates, as a resource of carbon, inorganic salts $MgSO_4x7H_2O$ (0,02%), $KH_2PO_4$ (0,2%), yeast autolysate (0,7%). The medium is adjusted to pH=6,5. Inoculum development time is 48 hours and inoculation of the main fermentor is with 5% inoculum. Fermentation is carried out for 120-168 hours at 25-28˚C with stirring and aeration. Fermented mass is diluted with water and separated on Westwalia separator with n=12000 degrees/min. Clear aqueous pullulan solution is precipitated with stirring, with 95% vol. ethanol to 50% vol. of alcohol in the mixture. Crude pullulan is precipitated after staying for 2 hours, by decanting separated from the supernatant, dissolved in water again, decoloured with active charcoal, and precipitated in the same manner as the ferment mass. Obtained crude pullulan has molar mass in range of $3x10^5$ - $4x10^6$ g/mol, intrinsic viscosity [η]=0,5-2,0 dl/g.

**EXAMPLE 2: Preparation of pullulan depolymerizates**

Crude pullulan aqueous solution, concentration 5-25% w/v is heated in the three-necked flask under reflux in water bath to working temperature (65-85˚C). After the tempeature is achieved, with continuous work of stirer, HCl (concentration of 200 $g/dm^3$) is added to achieve wanted pH. Depolymerization is carried out for 40-180 minutes, depending on wanted molar mass, neutralized with aqueous base solution, NaOH (concentration of 220 $g/dm^3$), and cool to the room temperature.

Obtained depolymerisate of pullulan is fractionally precipitated by 95% vol. ethanol in order to remove high molar masses with 38-42% vol. ethanol in the mixture, high fraction in the form of viscous precipitate is separated by decanting and the main fraction, suitable for synthesis, is precipitated with 65-75% vol. in the mixture. Yield of the fraction suitable for synthesis with ferric hydroxide is around 75-85% in regard to crude pullulan.

On Figure 3 FTIR spectra of depolymerized hydrogenated pullulan (a) and partially deuterated (b) used in this invention, are shown.

**EXAMPLE 3: Preparation of $\beta_2$-form of iron(III)-hydroxide**

To aqueous $FeCl_3x6H_2O$ solution, concentration 20% w/v, $Na_2CO_3$ solution, concentration 8% w/v, is dropwise added from the burrete, with intensive stirring during half an hour, to achieve pH of the suspension 6-6,5. Thich aqueous suspension is diluted, with stirring, with distilled water, and then, with centrifuging at 3000 degrees/min during 5 minutes, or filtration under vacuum, precipitate of iron(III)-hydroxyde is separated. In order to free the precipitate from salts, washing with distilled water is carried out to negative reaction on chlor ions (0,1% $AgNO_3$ solution).

Obtained precipitate of iron(III)-hydroxyde is identified by FTIR- spectroscopy, and the spectrum is shown on Figure 2, enclosed.

**EXAMPLE 4: Modification of pullulan by hydrogenation**

Pullulan fractions suitable for complexation with ir on(III)-hydroxyde, with molar mass in range of 6.000-12.000 is hydrogenated with $NaBH_4$. Amount of $NaBH_4$ needed for reaction (Q) depends on content of present reduction groups (RG) and amount of the pullulan hydrogenated (G).

Content of reduction groups is determined by the Somogyi method of titration. For calculation of needed quantity of $NaBH_4$ own empirical formula (Equation III) was used:

$$Q=0,95x10^{-3} \cdot RG(\%) \cdot G\ pul.(g) \qquad III$$

Needed quantity of $NaBH_4$ is in small portions, with continuous stirring, at temperature of pullulan solution of 40˚C, added, along with release of hydrogen, during 2 hours. Afterwards, the temperature of the reaction mixture is rised up to 70˚C, the mixture is intensively stirred in order to remove hydrogen from the solution, for 30 minutes. During hydrogenation, pH of the reaction mixture rises to 9-10, and the mixture is after the reaction is finished, adjus with diluted HCl solution to pH=6,0. In order to remove borates, the solution is dialyzed or passed through ion-exchange resins (to the negative reaction on borates).

Hydrogenated pullulan solution can be concentrated by evaporation, and directly used in synthesis, and can also be precipitated to 80% vol. ethanol in mixture with 95% vol. ethanol. Obtained hydrogenated pullulan must contain less than 0,05% of reduction groups.

**EXAMPLE 5: Synthesis of the complex with pullulan of molar mass 10278**

Depolymerizate of pullulan, with molar mass of 10278; intrinsic viscosity [η]=0,091dl/g and with contents of reducing sugars of 0,03% after hydration with $NaBH_4$, obtained in the previous example, is mixed with prepared gel of ferric hydroxide ($\beta_2$-FeOOH) in quantitative ratio iron : pullulan=1 : 3. The mixture is homogenized with stirring at temperature of 40˚C, and then solution of NaOH (10%) is used to adjust pH of synthesis to 10,5. Roughly homogenized mixture is then heated in an autoclave to 120˚C for 4 hours under pressure of $1,2x10^5$Pa. After reaction is completed, the obtained complex is clear viscous dark red liquid, with small quantity of precipitate, with pH lowered to 10,1. Subsequent processing of the complex, including evaporation and purification of the complex, results in preparation containing 26 mg/ml of Fe(III) and with an amount of pullulan of 68,7 mg/ml of preparation, with [η]=0,098 g/mol.

**EXAMPLE 6: Hydrogenated fraction**

Depolymerizate of pullulan from the previous example is mixed with prepared gel of ferric hydroxide ($\beta_2$-FeOOH) in the same concentration ratio iron : pullulan = 1 : 3. The mixture is homogenized with stirring at temperature of 40˚C for half an hour, and subsequently adjust pH of synthesis at 8,5 with NaOH solution, and roughly homogenized mixture is in an autoclave heated at temperature higher than 120˚C, pressure above $1,2\times10^5$Pa for three hours. After the reaction is completed, obtained complex is characterized with lowered pH of 9,98. Obtained complex is a clear, dark red solution, without precipitate, with iron content of 36,3 mg/ml and pullulan content of 81,2 mg/ml after evaporation.

**EXAMPLE 7: Synthesis of the complex with pullulan of molar mass 7096**

Depolymerizates of pullulan with molar mass 7096, intrinsic viscosity [η]=0,069 dl/g and with content of reducing sugars of 0,018% after hydrogenation with $NaBH_4$, are mixed with prepared gel of ferric hydroxide in concentracion ratios given in Table 1. All prepared mixtures are, first of all, roughly homogenized, at temperature of 40˚C for a while, and then, pH of synthesis is adjusted to 10,5 with 10%NaOH solution. Roughly homogenized mixtures are heated to boiling at increased temperature and under pressure for 4 hours. After the synthesis is completed, obtained complexes are clear, dark red solutions with small quantities of precipitate; content of iron of 5,585mg/ml, for the first sample, 10,89mg/ml, for the second sample, and 10,3mg/ml for the third sample. Using antron method content of pullulan in complexes is also determined (Table 2).

Table2: Content of iron(III) in complexes with the same ligand molar mass, and different concentration ratio of pullulan and iron(III) at the beginning of synthesis

| SAMPLE | $M_w$ of depolymerizate g/mol | [η] dl/g | concentration ratio $Fe^{3+}$: Pullulan before synthesis | pH of synthesis | content of $Fe^{3+}$in the complex, after completed synthesis mg/ml | content of pullulan in the compex mg/ml |
|---|---|---|---|---|---|---|
| complex 1 | 7096 | 0,069 | 1:2 | 10.5 | 5,58 | 7,75 |
| complex 2 | 7096 | 0,069 | 1:3 | 10.5 | 10,98 | 22,40 |
| complex 3 | 7096 | 0,069 | 1:5 | 10.5 | 10,30 | 39,40 |

**EXAMPLE 8: Synthesis of the complex with pullulan of molar mass 4100**

Depolymerizate of pullulan 4100 with intrinsic viscosity [η]=0,045dl/g, is hydrated, so that the content of reducing sugars is lower than 0,05%; it is mixed with prepared gel of ferric hydroxide in precisely determined concentration ratio iron : pullulan = 1 : 3. The mixture is, at first, roughly homogenized with stirring at lower temperature for a certain time, and then, using solution of a suitable base, pH of synthesis is adjusted to 10,5. Roughly homogenized mixture is subsequently complexed at temperature above 100˚C for 4 hours. After the reaction is completed, obtained complex is characterized with lowered pH of 9,7. Obtained complex is clear, dark red solution without precipitate, with content of iron of 52,3 mg/ml after evaporation, and content of pullulan of 150 mg/ml in the complex.

**EXAMPLE 9: Complex iron-pullulan**

Iron(III)- pullulan aqueous solution with iron content of 46,64 mg/ml passed the test of acute systemic toxicity, and $LD_{50}$ was determined. The test was carried out on white mice, with body weight in range 18-26 g, of both sexes, in groups of 6 members. The test was carried out according to Litchfield-Wilcoxon method. Intravenous application was carried out, and the results are shown in Table 3.

Table 3: Results of examination of acute systemic toxicity of the complex iron(III)-pullulan

| doses of preparation **mg/kg** | 863 | 1100 | 1150 | 1166 | 1255 | 1306 | 1350 | 1400 | 1464 | 1492 | 1586 | 1652 |
|---|---|---|---|---|---|---|---|---|---|---|---|---|
| **$LD_{50}$, %** | 0 | 0 | 0 | 0 | 0 | 33 | 33 | 33 | 66 | 66 | 100 | 100 |

From given results, average lethal dose of the examined preparation iron(III)-pullulan (46, 64 mg/ml) is LDso = 1429 mg/kg of body weight.

**EXAMPLE 10: Determination of complex iron-pullulan resorption**

Aqueous solution of iron(III)- pullulan with iron content of 46,64 mg/ml, passed the test of resorption of $Fe^{3+}$. As experimental animals, albino rabbits with body weight 2-3 kg, of both sexes, in groups of four members were used. We worked according to the method for examination of iron(III) from Fedex (A.D. »Zdravlje« Leskovac). The results are shown in Table 4:

Table 4: Results of examination of the complex iron(III)-pullulan resorption

| **Experimental animals** | **serum before application** | **serum 24 h after application** | **serum 36 h after application** |
|---|---|---|---|
| 1. rabbit 3250 g | 0,9114 mg/ml | 9,491 mg/ml | 10,069 mg/100ml |
| 2. rabbit 3000 g | 1,1890 mg/ml | 11,077 mg/ml | 10,404 mg/100ml |
| 1. rabbit 2900 g | 1,2029 mg/ml | 13,654 mg/ml | 11,278 mg/100 ml |

(continued)

| Experimental animals | serum before application | serum 24 h after application | serum 36 h after application |
|---|---|---|---|
| 1. rabbit 3300 g | 1,0548 mg/ml | 8,160 mg/ml | 8,033 mg/100ml |

From the presented results it can be concluded that the peak resorption of iron(III) is 13,564mg/100ml.

**EXAMPLE 11: Day light influence on the complex stability**

Aqueous solution of iron(III)-pullulan with iron content of 46,64 mg/ml is examined in regard to influence of day light at room temperature (20˚C). The results are shown in Table 5.

Table 5: Influence of light on stability of complex iron(III)-pullulan

| time of sampling | colour of solution | quantity of $Fe^{3+}$in the complex, mg/ml | content of pullulan in the complex, mg/ml | present precipitate, mg |
|---|---|---|---|---|
| 0 min | dark red | 46,64 | 121,3 | no precipitate |
| 5 min | dark red | 46,64 | 120,9 | no precipitate |
| 15 min | dark red | 46,60 | 121,3 | no precipitate |
| 30 min | dark red | 46,62 | 121,3 | no precipitate |
| 60 min | dark red | 46,64 | 120,9 | no precipitate |
| 90 min | dark red | 46,23 | 121,3 | no precipitate |
| 150 min | dark red | 46,60 | 121,3 | no precipitate |
| 210 min | dark red | 46,61 | 121,3 | no precipitate |
| $5^h$ | dark red | 46,30 | 120,9 | no precipitate |
| $12^h$ | dark red | 46,65 | 121,0 | no precipitate |
| $24^h$ | dark red | 46,63 | 121,3 | no precipitate |
| $48^h$ | dark red | 46,24 | 121,3 | no precipitate |
| 5 days | dark red | 46,61 | 121,0 | no precipitate |
| 15 days | dark red | 46,35 | 121,3 | no precipitate |
| 30 days | dark red | 46,61 | 121,3 | no precipitate |
| 2 months | dark red | 46,62 | 121,0 | no precipitate |
| 4 months | dark red | 46,63 | 121,3 | no precipitate |

In regard to the starting sample, examinations did not show any changes in contents of iron(III) and pullulan in complex, also, nither gelling of the complex was noticed, nor change in colour, nor appearance of precipitate, which leads to conclusion that the preparation is stable regarding day light.

**EXAMPLE 12: Examination of the preparation stability at temperature changes**

Aqueos solution of iron(III)-pullulan with iron content of 46,64 mg/ml is examined in regard to influence of temperature. The samples were examined at +4˚C (they were kept in refrigerator), at room temperature (20˚C) and in the chamber at 45˚C, 70% humidity. The results of examinations are given in Table 6.

Table 6: Influence of temperature on stability of complex iron(III)-pullulan

| time of sampling | content of $Fe^{3+}$at +4˚C, mg/ml | content of $Fe^{3+}$at 20˚C, mg/ml | content of $Fe^{3+}$ at 45˚C, mg/ml | content of pullulan at +4˚C, mg/ml | content of pullulan at +20˚C, mg/ml | content of pullulan at +45˚C, mg/ml | colour of complex |
|---|---|---|---|---|---|---|---|
| 0 min | 46,64 | 46,64 | 46,64 | 121,0 | 121,0 | 121,0 | dark red |
| 5 min | 46,63 | 46,62 | 46,62 | 120,3 | 120,1 | 120,3 | dark red |
| 15 min | 46,62 | 46,60 | 46,61 | 121,3 | 121,3 | 121,3 | dark red |
| 30 min | 46,62 | 46,62 | 46,64 | 121,0 | 121,0 | 121,0 | dark red |
| 60 min | 46,61 | 46,60 | 46,65 | 120,8 | 121,3 | 121,1 | dark red |
| 90 min | 46,34 | 46,53 | 46,63 | 121,3 | 121,3 | 121,3 | dark red |
| 160 min | 46,61 | 46,61 | 46,61 | 120,9 | 120,9 | 120,7 | dark red |
| 220 min | 46,61 | 46,61 | 46,61 | 121,3 | 121,1 | 121,2 | dark red |
| $5^h$ | 46,60 | 46,62 | 46,60 | 121,0 | 121,1 | 121,3 | dark red |
| $12^h$ | 46,64 | 46,64 | 46,63 | 120,8 | 121,1 | 120,9 | dark red |
| $24^h$ | 46,63 | 46,63 | 46,63 | 120,6 | 121,0 | 121,2 | dark red |
| $48^h$ | 46,29 | 46,24 | 46,20 | 121,1 | 120,3 | 121,0 | dark red |
| 5 days | 46,46 | 46,61 | 46,63 | 121,1 | 121,2 | 121,2 | dark red |
| 15 days | 46,60 | 46,58 | 46,55 | 120,9 | 120,9 | 120,9 | dark red |
| 30 days | 46,61 | 46,61 | 46,61 | 121,2 | 121,1 | 121,2 | dark red |
| 2 months | 46,63 | 46,62 | 46,64 | 121,3 | 121,3 | 121,3 | dark red |
| 4 months | 46,64 | 46,63 | 46,64 | 121,3 | 121,3 | 121,3 | dark red |

The results of examination of influence of temperature on stability of the complex, show that after four months, physical and chemical characteristics of the complex remained unchanged (appearance of small disagreements is the result of experimental error).

**EXAMPLE 13: Influence of hydrolytic effect on the complex stability**

Stability of the complex in regard to hydrolytic effect of HCl is determined by time necessary for complete converting of iron from complex into ferric chloride. To the complex sample (1 $cm^3$), in an Erlenmeyer flask, 20 $cm^3$ of distilled water, and 15 $cm^3$ of concentrated HCl are added, the mixture is gently shaken, and time of hydrolysis is measured starting from the moment of addition of the acid, to complete turning of brown colour of the solution into light-yellow nuance. Time of hydrolysis for the sample containing iron(III) in amount of 46,64 mg/ml is 5 seconds.

**EXAMPLE 14: Influence of aerous oxygen on the complex stability**

Aqueous solution of the complex iron(III)-pullulan with iron content of 46,64 mg/ml is brought together with air oxygen. The samples for examination of content of iron(III) and carbohydrates in the complex are taken in regular intervals, and they are shown in Table 7.

Table 7: Influence of air oxygen on stability of complex iron(in)-pullulan

| time of sampling | colour of solution | quantity of $Fe^{3+}$in the complex, mg/ml | content of pullulan in the complex, mg/ml | present precipitate, mg |
|---|---|---|---|---|
| 0 min | dark red | 46,64 | 121,3 | no precipitate |
| 5 min | dark red | 46,64 | 120,9 | no precipitate |
| 15 min | dark red | 46,60 | 121,3 | no precipitate |

(continued)

| time of sampling | colour of solution | quantity of $Fe^{3+}$in the complex, mg/ml | content of pullulan in the complex, mg/ml | present precipitate, mg |
|---|---|---|---|---|
| 30 min | dark red | 46,62 | 121,3 | no precipitate |
| 60 min | dark red | 46,64 | 120,9 | no precipitate |
| 90 min | dark red | 46,23 | 121,3 | no precipitate |
| 150 min | dark red | 46,60 | 121,3 | no precipitate |
| 210 min | dark red | 46,61 | 121,3 | no precipitate |
| $5^h$ | dark red | 46,30 | 120,9 | no precipitate |
| $12^h$ | dark red | 46,65 | 121,0 | no precipitate |
| $24^h$ | dark red | 46,63 | 121,3 | no precipitate |
| $48^h$ | dark red | 46,24 | 121,3 | no precipitate |
| 5 days | dark red | 46,61 | 121,0 | no precipitate |
| 15 days | dark red | 46,35 | 121,3 | no precipitate |
| 30 days | dark red | 46,61 | 121,3 | no precipitate |
| 2 months | dark red | 46,62 | 121,0 | no precipitate |
| 4 months | dark red | 46,63 | 121,3 | no precipitate |

During 4 months of these examinations, the colour of the solution remained the same, no precipitation as a result of decomposition of the complex appeared, contents of iron(III) and carbohydrates in the complex remained unchanged.

**EXAMPLE 15: Production of injection preparation**

For obtaining of injectable peparation, it is necessary to carry out processing of polynuclear iron(III)-complex with oligomers of pullulan, in following way: first of all determine contents of iron and carbohydrates and concentration ratio between them, and then, adjust the iron(III) content to 50 mg/ml for human use, and 75 mg/ml and 100 mg/ml for veterinary use; determine concentration ratio pullulan: iron(III), adjust contents of chlorides to an isotonic solution at 0,9%, add phenol as a preservative 0,5%, adjust pH of preparation in range 5,2 to 6,5, carry out thermal sterilization in an autoclave for 20 minutes at 120˚C and under pressure of $1,013x10^5$Pa. After physical and chemical standardization, the preparation is packed in ampullas.

## Claims

**1.** A polynuclear complex of pullulan oligomers of formula (I):

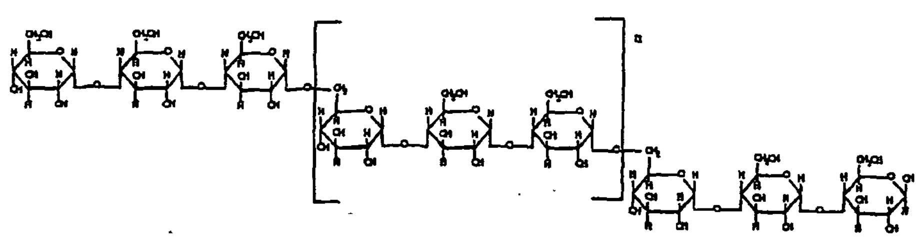

(I)

having a Mw in the range of 1000 to 25000 with iron(III)-hydroxide of formula (II):

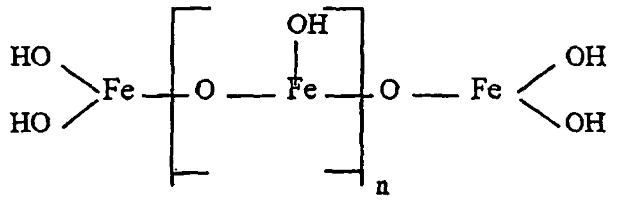

(II)

wherein n = 900.

2. Polynuclear complex of pullulan oligomers with iron(III)-hydroxyde, according to claim 1 marked by the fact, that the complex particles have approximately spherical shape.

3. Polynuclear complex of pullulan oligomers with iron(III)-hydroxyde according to claim 1 or 2 marked by the fact, that the complex particles have an average size of 6-25 nm, composed of iron(III)-hydroxyde with an average particle size of 3-18 nm.

4. A polynuclear complex of pullulan oligomers with iron(III)-hydroxide according to any of claims 1 to 3 marked by the fact that it is a viscous, dark red solution, stable at temperatures in range +4 to +50˚C, stable regarding light and air oxygen, with time of hydrolytic decomposition of the complex with HCl in range 5 to 7 seconds.

5. A polynuclear complex of pullulan oligomers with iron(III)-hydroxide, according to patent claims 1, 2, 3 and 4, for application as a medication in prevention and therapy of syderopenic anemia in human and veterinary medicine.

6. A process for obtaining of the complex, marked by the fact that the native pullulan oligomers react with iron(III)-hydroxyde at rised temperature and definite pressure in order to obtain polynuclear complex of pullulan oligomers with iron(III)-hydroxide.

7. A process for obtaining of polynuclear complex of pullulan oligomers with iron(III)-hydroxide, according to the patent claim 6, **marked by the fact that** it is carried ont using the process of synthesis of native pollulan oligomers, previously depolymerized with mineral acids, subsequently hydrogenated with a suitable hydrogenating agent, or oxidated with an oxidative agent, with pH adjusted before the synthesis starts, which is put into reaction with synthetized Iron(III)-hydroxide gel, according to the assigned concentration ratio, so that the mixture is, first of all, roughly homogenized with stirring at temperature helow 60˚C, and then, finely homogenized at temperature above 80˚C and under pressure; after the synthesis is completed, pil is adjusted, and subsequently the mixture is purified by passing through ion exchange resins, filtered through Seitz filter, and evaporated in vacuum evaporator to certain volume.

8. A process for obtaining of polynuclear complex of pullulan with iron(III)-hydroxide, according to the claim 6, **marked by the fact that** the temperature of synthesis is 80 to 250˚C primarily in range 100 to 160˚C, and pressure is in range $1,013x10^5$Pa to $4,5x10^5$Pa, primarily $1,013x10^5$ to $2,3x10^5$Pa.

9. A process according to the claim 6, **marked by the fact that** for synthesis of the complex, pullulan with molar mass $M_w$ in range 1000 to 25000 and intrinsic viscosity [η] in range 0,01 to 0,16 dl/g, primarily with molar weight in range 3000 to 12000 and intrinsic viscosity [η] = 0,028 dl/g 0,078 dl/g.

10. A process for obtainig of complex, according to the patent claim 6 **marked by the fact that** ph of synthesis of complex must be between 7 and 12, primarily in range 9,5 to 11, adjusted with bases such as: KOH, $Ca(OH)_2$, most frequently NaOH.

11. A process for obtainig of complex, according to the patent claim 6, **marked by the fact that** pullulan oligomer, obtained by procedure of acid depolymerization, previously hydrogenated, or oxidized with suitable agents, is used for synthesis, so that the amount of reducing sugars in it is lower than 0,05%, in order to achieve limit molar mass necessary for binding with ferric-hydroxide in the complex.

12. A process according to the claim 6, **marked by the fact that** purification of aqueous solution of complex iron(III) with pullulan oligomers is carried out by passing through the column with ion-exchange resins Amberlit IRA-120 as a cation exchanger and Amberlit IRA-410 as an anion exchanger.

**13.** A process for synthesis of complex, according to the patent claim 6, **marked by the fact that** it shows concentration ratio iron(III) : pullulan 1:2 to 1:5, primarily ratio iron(III) pullulan 1:3.

**14.** A process for obtaining of complex, according to the patent claim 6, **marked by the fact that** obtained polynuclear complex of pullulan and iron is **characterized by** iron content of 5,585 mg/ml to 100 mg/ml and pullulan content of 15 mg/ml to 500 mg/ml.

**15.** A process for obtaining of complex, according to the patent claim 6, **marked by the fact that** a process for depolymerization of pullulan for synthesis is carried out primarily by acid hydrolysis with hydrochloric acid at temperature between 60 and 95˚C and time of hydrolysis in range 30 to 280 minutes, then, depolymerizates are further codified by partial reduction with catalysts; $LiAlH_4$, or $NaBH_4$ within the process of catalytic hydrogenation or oxidation by treating with oxidizing agents such as bromine, sodium hypobromite, sodium bromite, sodium hypochlorite, sodium chlorite, and sodium periodate, resulting with depolymerisates with an average molar mass in range 1000 to 25000, most frequently 3000 to 12000.

**16.** Use of polynuclear complex, according to patent claims 1 to 5, for obtaining of pharmaceutical preparation for prevention and therapy of syderopenic anemia in human and veterinary medicine.

**17.** Pharmaceutical preparations for human and veterinary use containing polynuclear complex of pullulan oligomers with iron(III)-hydroxide, according to patent claim 1, and usual additives in order to prepare them in form suitable for pharmaceutical use.

**18.** A pharmaceutical preparation according to claim 17, for prevention and therapy of sideropenic anemia, with iron content 50 mg/ml as a human preparation that is with iron content of 75 mg/ml. and 100 mg/ml for veterinary use.

**19.** An injectible preparation containing polynuclear complex of pullulan oligomers with iron(III). hydroxide, **marked by the fact that** processing of polynuclear complex of iron(III) with pullulan oligomers is carried out by determination of contents of iron(III) and pullulan, concentration ratio between them, adjusting of content of iron to 50 mg/ml$Fe^3$ (for human use) and pullulan according to desired concentration ratio, and to 75 $Fe^{3+}$ mg/ml and 100 $Fe^{3+}$ mg/ml for veterinary use; subsequently, a suitable preservative in concentration of 0,5%, most frequently phenoles and chlorides, is added using NaCl to concentration of 0,9%, pH is adjusted to 5,2 to 6,5 and then, thermal sterilization in an autoclave for 20 minutes, at 120˚C and under pressure of $1,013x10^5$Pa is carried out, and the preparation is, finally, packed in ampullas.

**Patentansprüche**

**1.** Polynuklealer Komplex von Pululan Oligomeren mit der Formel (I) :

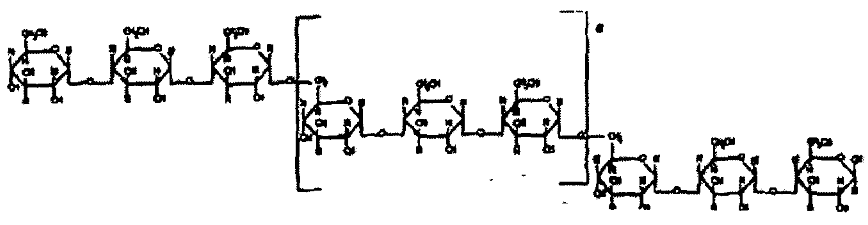

der Mw in einem Umfang von 1000 bis 25000 Eisen (III) Hydroxid enthält, mit der folgenden Formel (II) :

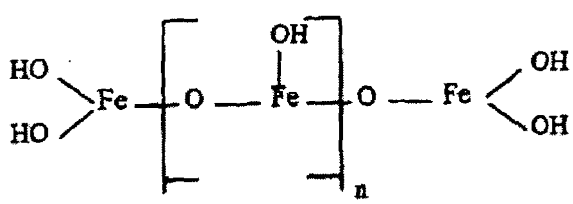

und n = 900

**2.** Polynuklealer Komplex von Pululan Oligomeren mit Eisen (III) Hydroxid im Einklang mit dem Antrag auf Patent 1, **der dadurch gekennzeichnet wird,** dass die Komplexteile eine annähernd sphärische Form haben.

**3.** Polynuklealer Komplex von Pululan Oligomeren mit Eisen (III) Hydroxid im Einklang mit dem Antrag auf Patent 1 oder 2, der **dadurch gekennzeichnet** wird, dass die Komplexteile eine mittlere Größe von 6 - 25 nm haben und **dadurch** dass er aus Eisen (III) Hydroxid mit einer mittleren Größe der Partikeln von 3 - 18 nm zusammengestellt ist.

**4.** Polynuklealer Komplex von Pululan Oligomeren mit Eisen (III) Hydroxid im Einklang mit irgendwelchen Antrag auf Patent von 1 bis 3, der **dadurch gekennzeichnet** wird, dass es sich hier um eine viskose, dunkel-rote Lösung handelt, die sowohl bei Temperaturen von + 4 bis + 50˚ C als auch bei Licht und Luft unverändert bleibt. Bei dem Komplex geschieht in 5 bis 7 Sekunden ein hydrolytischer Zerfall mit HCL.

**5.** Polynuklealer Komplex von Pululan Oligomeren mit Eisen (III) Hydroxid im Einklang mit den Anträgen 1, 2 , 3 und 4, der Anwendung als Arzneimittel zur Behandlung und zum Schutz der sideropenischen Anämie in der human- und veterinären Medizin findet.

**6.** Das Verfahren zum Gewinn dieses Komplexes wird **dadurch gekennzeichnet, dass** die natürlichen Pululan Oligomere mit Eisen (III) Hydroxid auf erhöhte Temperatur und bestimmten Druck reagieren, mit dem Ziel einen polynuklearen Komplex von Oligomeren mit Eisen (III) Hydroxid Pululan zu gewinnen.

**7.** Das Verfahren zum Gewinn dieses Komplexes von Pululan Oligomeren mit Eisen (III) Hydroxid wird, im Einklang mit dem Antrag 6, **dadurch gekennzeichnet, dass** das Verfahren zur Synthese von natürlichen Pululan Oligomeren, die vorher mit Mineralsäuren depolymerisiert , nach und nach mit entsprechend hydrogenisierender Agenz hydrogeniert oder mit oxidativer Agenz oxidiert sind, vor Anfang der Synthese mit pH angepasst , ausgedruckt durch die Reaktion mit synthetisiertem Eisen (III) Hydroxidgel , im Einklang mit dem vorgeschriebenen Verhältnis, so dass die Masse vor allem annähernd mit Mischung der Temperatur unter 60˚C und zum Schluss auf 80˚C und unter Druck homogenisiert ,durchgeführt wird. Nachdem die Synthese zu Ende gebracht wird, wird der pH-Wert angepasst und die Masse nach und nach gesäubert, indem sie sowohl durch ion-wechselnde Harze durchlaufen gelassen als auch durch Seitz-Filter filtriert und bis zum bestimmten Volumen mit Vakuum-Dämpfer aufgedämpft, durchgeführt wird.

**8.** Das Verfahren zum Gewinn dieses Komplexes von Pululan Oligomeren mit Eisen (III) Hydroxid **wird,** im Einklang mit dem Antrag 6, **dadurch gekennzeichnet, dass** die Temperatur der Synthese von 80 bis 250˚ C , primär von 100 bis 160˚ C, und der Druck vo 1.013 x $10^5$ Pa bis 4,5 x $10^5$ Pa, primär 1.013 x $10^5$ Pa bis 2,3 x $10^5$ Pa, beträgt.

**9.** Das Verfahren im Einklang mit dem Antrag 6 **wird durch** die Synthese des Komplexes, Pululan mit molarer Masse Mw von 1000 bis 25000 und durch die intrinsische Viskosität [0] von 0.01 bis 0.16 dl/g, durch das primäre molare Gewicht von 3000 bis 12000 und die intrinsische Viskosität [0]= 0.028 dl/g-0.078 dl/g **gekennzeichnet**.

**10.** Das Verfahren zum Gewinn dieses Komplexes, im Einklang mit dem Antrag 6, wird **dadurch gekennzeichnet, dass** bei der Synthese des Komplexes der pH-Wert zwischen 7 und 15 liegen muss, primär von 9,5 bis 11, zu den Basen wie KOH, $Ca(OH)_2$ meistens zu NaOH angepasst.

**11.** Das Verfahren zum Gewinn dieses Komplexes, im Einklang mit dem Antrag 6, wird **dadurch gekennzeichnet, dass** das Pululan Oligomer, das durch das Verfahren der sauren Depolymerisation gewonnen und vorher mit entprechenden Agenzen hydrogenisiert oder oxidiert wurde, für die Synthese verwendet wurde, so dass seine Menge von Zucker weniger als 0.05% beträgt und man eine beschränkte Molarmasse, die zur Verbindung mit dem Eisenhydroxid im Komplex nötig ist, erreichen kann.

**12.** Das Verfahren im Einklang mit dem Antrag 6 wird **dadurch gekennzechnet, dass** die Säuberung der wässrigen Lösung vom komplexen Eisen (III) mit Pululan Oligomeren durch das Durchlaufen durch die ion-wechselnde Harze Amberlit IRA-120, als kationer Veränderer, und durch Amberlit IRA-410 , als anioner Veränderer durchgeführt wird.

**13.** Das Verfahren zur Synthese des Komplexes im Einklang mit dem Antrag 6 **wird dadurch gekennzechnet, dass** es ein Verhältnis der Eisenkonzentration (III) : Pululan 1:2 bis 1:5 zeigt, primär Eisenkonzentration (III) Pululan 1:3.

**14.** Das Verfahren zum Gewinn des Komplexes, im Einklang mit dem Antrag 6, **wird dadurch gekennzechnet, dass** sich der gewonnene polynukleare Komplex von Pululan und Eisen mit einem Gehalt von 5,585 mg/ml an Eisen und von einem Gehalt von 15mg/ml bis 500mg/ml an Pululan charakterisiert.

**15.** Das Verfahren zum Gewinn des Komplexes, im Einklang mit dem Antrag 6, **wird dadurch gekennzechnet**, dass das Verfahren zur Depolymerisierung des Pulpulans zur Synthese primär durch säuerliche Hydrolyse mit Chlorwasserstoffsäure auf einer Temperatur zwischen 60˚ und 90˚ in einem Zeitraum der Hydrolyse von 30 bis 280 Minuten durchzuführen ist. Die Depolymerisate werden dann weiter durch partielle Reduktion mit Kathalisatoren modifiziert : $LiAlH_4$ oder $NaBH_4$ beim Verfahren der katlitischen Hydrogenisierung oder Oxidierung durch Behandlung mit oxidierenden Agenzen wie : Brom, Natriumhypobromid, Natriumbromid, Natriumhypochlorid, Natriumchlorid und Natriumperjodat, die zu Depolymerisaten mit durchschnittlicher Molarmasse von 1.000 bis 25.000, meistens von 3.000 bis 12.000 führen.

**16.** Anwendung von polynuklearem Komplex, im Einklang mit den Anträgen 1 bis 5, zum Gewinn von pharmazeutischem Mittel zur Vorbeugung und Behandlung in der human-veterinären Medizin.

**17.** Pharmazeutische Mittel zur Anwendung bei Menschen und Tieren enthalten einen polynuklearen Komplex von Pululan Oligomeren mit Eisen (III) Hydroxid, im Einklang mit dem Antrag auf Patent 1; und gewöhnlichen Inhalten mit dem Ziel sie in ensprechender Form zur pharmazeutischen Anwendung vorzubereiten.

**18.** Pharmazeutisches Mittel im Einklang mit dem Antrag auf Patent 17 zur Vorbeugung und Behandlung der sideropenischen Anämie mit einem Gehalt von 50mg/ml an Eisen für Menschen und mit einem Gehalt von 75mg/ml und 100mg/ml an Eisen für Tiere.

**19.** Injektionsmittel, das einen polynuklearen Komplex von Pululan Oligomeren mit Eisen (III) Hydroxid enthält, **wird wie folgt gekennzechnet:** Die Prozessuierung des polynuklearen Komplexes von Eisen (III) Hydroxid mit Pululan Oligomeren ist mit Inhaltsbestimmung von Eisen (III) und Pululan und mit Bestimmung deren Konzentration, mit Anpassung des Eisengehaltes bis zum 50 mg/ml $Fe^{3+}$ (zur Anwendung an Menschen) und Pululan im Enklang mit dem gewünschten Konzentrationsverhältnis, und bis 75 mg/ml $Fe^{3+}$ und 100 mg/ml $Fe^{3+}$ zur Anwendung an Tieren, durchgeführt. Zur vetärineren Anwendung ist nach und nach entsprechender Konservierungsstoff in einer Konzentration von 0,5%, meistens Phenole und Chloride und NaCl bis zu einer Konzentration von 0,9%, zugegeben. Der pH-Wert ist von 5,2 bis 6,5 angepasst, es ist auch eine Wärmesterilisirung in einem Autoklav in Dauer von 20 Minuten auf einer Temperatur von 120˚C und unter Druck von 1,013 x $10^5$ Pa durchgeführt worden. Zum Schluss ist das Präparat in Ampulen gefüllt.

## Revendications

**1.** Complexe polynucléaire d'oligomères pullulans ayant la formule (I):

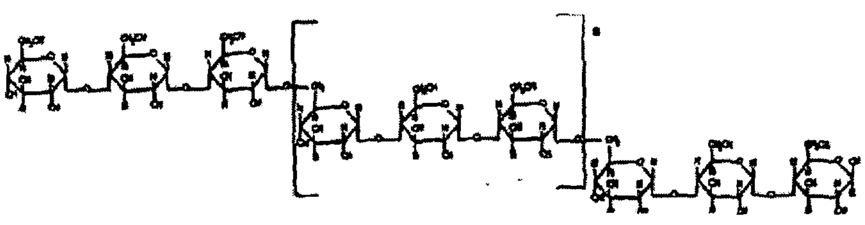

(I)

contenant Mw pouvant varier entre 1000+++ et 25000 du fer (III) hydroxyde ayant la formule (II):

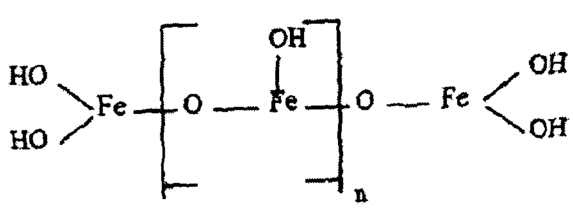

où n=900

2. Le complexe polynucléaire d'oligomères pullulans avec du fer (III) hydroxyde selon la revendication 1, **caractérisé en ce que** les parties du complexe ont une forme approximativement sphérique.

3. Le complexe polynucléaire d'oligomères pullulans avec du fer (III) hydroxyde selon la revendication 1 ou 2, **caractérisé en ce que** les parties du complexe ont une taille moyenne de 6-25 nm, composé du fer (III) hydroxyde avec une taille moyenne des particules entre 3-18 nm.

4. Le complexe polynucléaire d'oligomères pullulans avec du fer (III) hydroxyde, conformément à n'importe quelle revendication de 1 à 3, **caractérisé en ce que** il présente une solution viscose, rouge sombre, stable à une température allant de +4°C jusqu'à +50°C, stable à la lumière et à l'air, avec un temps de décomposition hydrolytique avec HCL entre 5 et 7 secondes.

5. Complexe polynucléaire d'oligomères pullulans avec du fer (III) hydroxyde, selon la revendication 1, 2, 3 et 4, pour usage en tant que médicament concernant la prévention et la thérapie de l'anémie ferriprive dans la médicine humaine et vétérinaire.

6. Procédé d'obtention de complexe, **caractérisé en ce que** les oligomères pullulans naturels réagissent avec du fer (III) hydroxyde à une température élevée et une pression définie à fin d'obtenir un complexe polynucléaire d'oligomères pullulans contenant du fer (III) hydroxyde.

7. Procédé d'obtention de complexe polynucléaire d'oligomères pullulans contenant du fer (III) hydroxyde, selon la revendication 6, **caractérisé en ce que** se déroule en utilisant la démarche de synthèse des oligomère pullulans naturels, préalablement dépolymérisés avec des acides minérales, graduellement hydrogénées avec un agent hydrogéniteur respectif ou oxygénées avec un agent oxydatif, avec pH adapté avant le commencement de la synthèse qui est exprimée dans la réaction avec du fer (III) gel hydroxyde synthétisé, conformément au rapport concentré proscrit, de manière que le mélange est avant tout homogénéisé par une mixtion à température au dessous de 80°C et sous pression, dès que la synthèse soit finie, la pH est adaptée et le mélange est progressivement purifié à travers le passage par des résines échangeant des ions, filtrées par un filtre de Seitz et évaporé par un évaporateur à vacuum jusqu'aux certains volumes.

8. Procédé d'obtention de complexe polynucléaire d'oligomères pollulans avec du fer (III) hydroxyde, selon la revendication 6, **caractérisé en ce que** la température de synthèse va de 80 jusqu'à 250°C primairement allant de 100 à 160°C et la pression allant de $1.013 \times 10^5$Pa à $4,5 \times 10^5$ Pa, primairement $1.013 \times 10^5$ ДО Pa $2,3 \times 10^5$ Pa.

9. Procédé selon la revendication 6, **caractérisé en ce que** la synthèse du complexe, pullulan ayant une masse moléculaire Mw allant du 1000 à 25000 et viscosité intrinsèque [η] de 0.01 à 0.16 dl/g, primairement ayant un poids moléculaire allant de 3000 à 12000 et une viscosité intrinsèque [η]=0.028 dl/g-0.078 dl/g.

10. Procédé d'obtention d'un complexe, selon la revendication 6, 1 **caractérisé en ce que** a pH pendant la synthèse du complexe doit être entre 7 et 12, primairement variant entre 9.5 et 11, adapté aux bases telles que: KOH, $Ca(OH)_2$, le plus souvent NaOH.

11. Procédé d'obtention de complexe, selon la revendication 6, **caractérisé en ce que** l'oligomère pullulan, obtenu par le procédé de dépolymérisation acide, préalablement hydrogéné ou oxygéné avec des agents correspondants, est utilisée pour une synthèse, de manière que la quantité des sucres réduits en lui sont moins de 0.05%, à fin d'atteindre une masse moléculaire limitée nécessaire pour une liaison avec le fer hydroxyde dans un complexe.

12. Procédé selon la revendication 6, **caractérisé en ce que** la purification de la solution aqueuse du complexe fer (III) avec des oligomères pullulans est réalisé par un passage à travers du pilier des résines échangeant des ions Amberlit IRA-120 en tant qu'échangeur de cations et Amberlit IRA-410 en tant qu'échangeur d'anions.

13. Procédé de synthèse de complexe, selon la revendication 6, **caractérisé en ce que** montre un rapport de concentration de fer (III): pullulan 1:2 jusqu'à 1:5, primairement rapport de concentration du fer (III) pullulan 1:3.

14. Procédé d'obtention de complexe, selon la revendication 6, **caractérisé en ce que** le complexe polynucléaire

obtenu de pullulan et de fer se **caractérise par** un contenu en fer de 5,585mg/ml à 100mg/ml et contenu en pullulan de 5mg/ml à 500mg/ml.

**15.** Procédé d'obtention de complexe selon la revendication 6, **caractérisé en ce que** le procédé de dépolymérisation de pullulan pour une synthèse est fait primairement à travers d'hydrolyse acide avec d'acide chlorhydrique à une température entre 60° et 95° et un temps de hydrolyse entre 30 et 280 minutes, puis, les dépolymérisables sont ensuite modifiés avec une réduction partielle à l'aide des catalyseurs: $LiAlH_4$ ou $NaBH_4$ dans le procédé d'hydrogénasse ou d'oxydation à travers un traitement avec des agents oxygénateurs tels que: brome, natrium hypobromide, natrium chloride et natrium periodate, résultant en dépolymerisateurs avec une masse moléculaire moyenne allant de 1.000 à 25.000, le plus souvent entre 3.000 et 12.000.

**16.** L'usage d'un complexe polynucléaire, selon la revendication 1 à 5 ; pour obtention d'une préparation pharmaceutique pour prévention et thérapie de l'anémie ferriprive dans la médecine humaine et vétérinaire.

**17.** Préparations pharmaceutiques pour un usage humaine et vétérinaire contenant un complexe polynucléaire d'oligomères pullulans avec du fer (III) hydroxyde, selon la revendication 1; et des compléments habituels à fin de les préparer sous une forme d'usage pharmaceutique.

**18.** Préparation pharmaceutique selon la revendication 17, pour prévention et thérapie de l'anémie ferriprive, avec un contenu en fer de 50mg/ml en tant que préparation humaine, qui a un contenu en fer de 75mg/ml et 100mg/ml pour un usage vétérinaire.

**19.** Préparation d'injection qui contient un complexe polynucléaire d'oligomères pullulans avec du fer (III) hydroxyde, **caractérisé en ce que** la transformation du complexe polynucléaire du fer (III) avec des oligomères est faite à travers la détermination du contenu en fer (III) et en pullulan, le rapport de concentration entre eux, l'ajustement du contenu en fer de 50 mg/ml$Fe^{3+}$ (pour usage humaine) et en pullulan selon le rapport désiré de concentration, et jusqu'à 75 mg/ml$Fe^{3+}$ et 100 mg/ml$Fe^{3+}$ pour un usage vétérinaire en ajoutant graduellement un conservant correspondant dans une concentration de 0,5%, le plus souvent phénols et chlorites, en utilisant NaCl jusqu'à une concentration de 0,9%, la pH est adaptée de 5,2 à 6,5, et ensuite une stérilisation thermale est réalisée dans un autoclave avec une durée de 20 minutes, à température de 120°C et sous pression de 1,013 x $10^5$Pa, et la préparation est finalement conditionnée dans des ampoules.

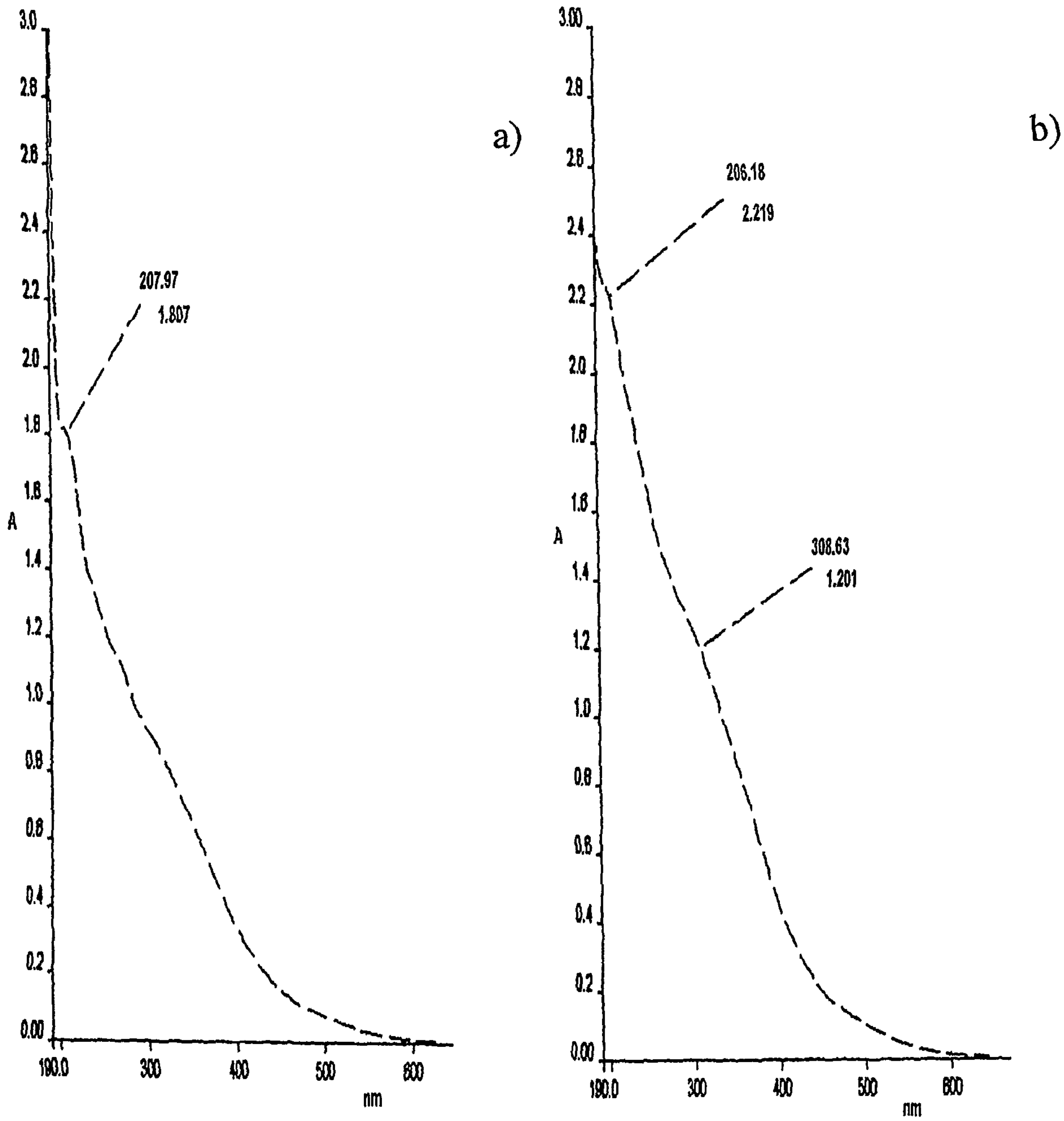


Figure 1: UV-VIS spectra of the iron(III) complex with oligomers of:
a): pullulan (pH=2, T=85°C, t=140 min);
b): dextran (pH=2,2, T=85°C, t=120min)

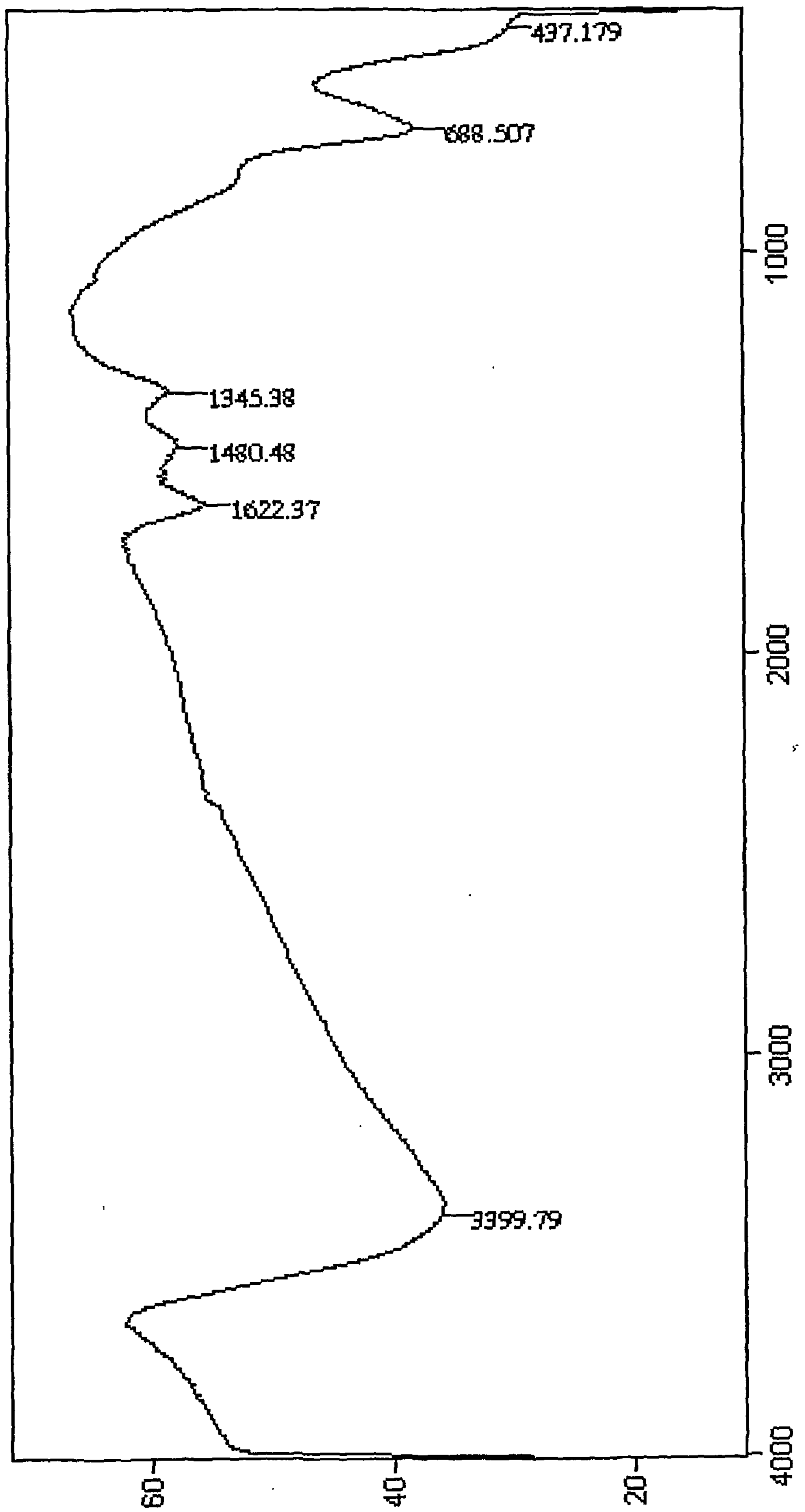


Figure 2: FTIR srectrum of polynuclear $\beta_2$-FeOOH

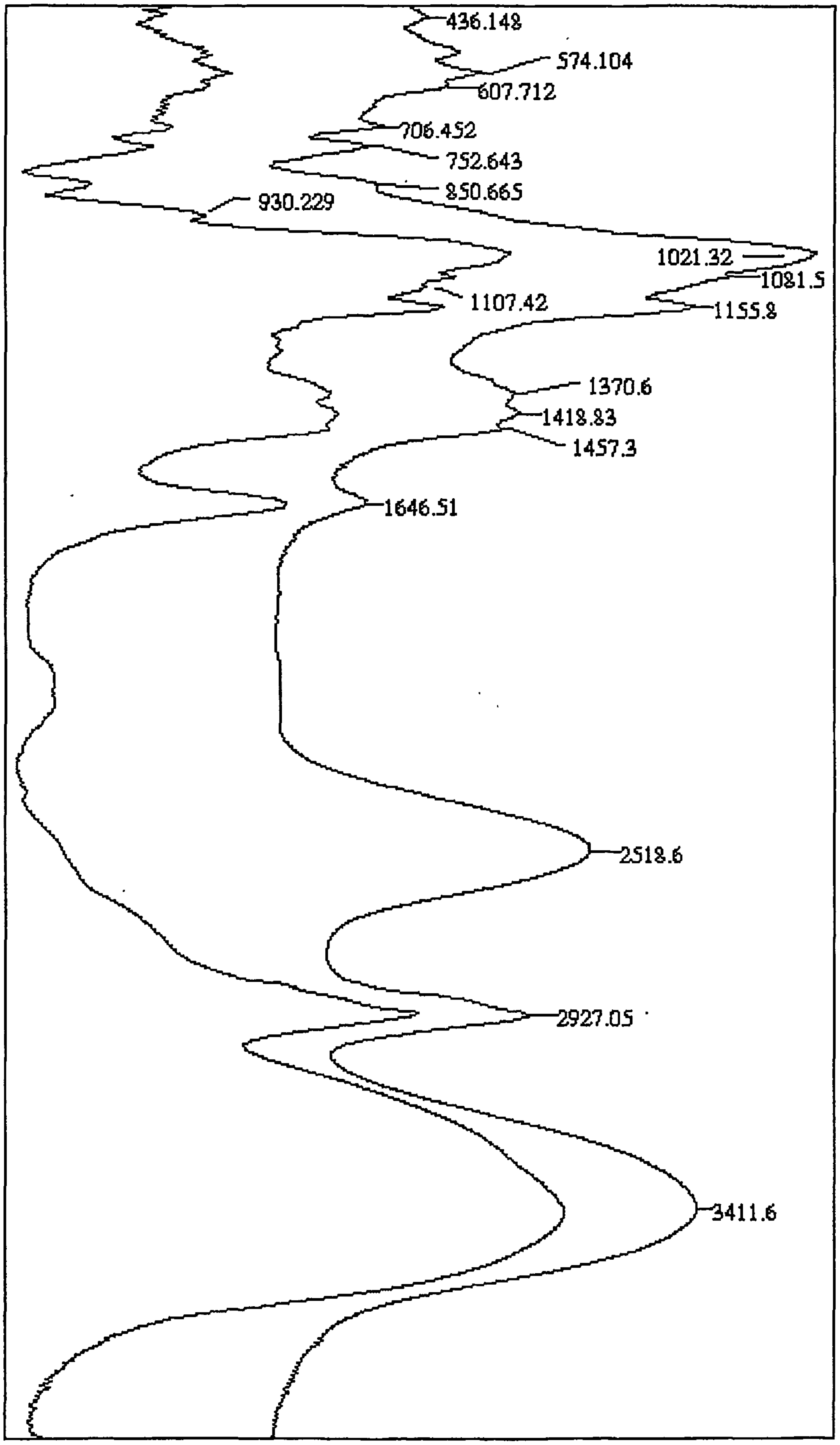


Figure 3: FTIR spectra: hydrogenated pullulan (a), partially deuterated pullulan (b)

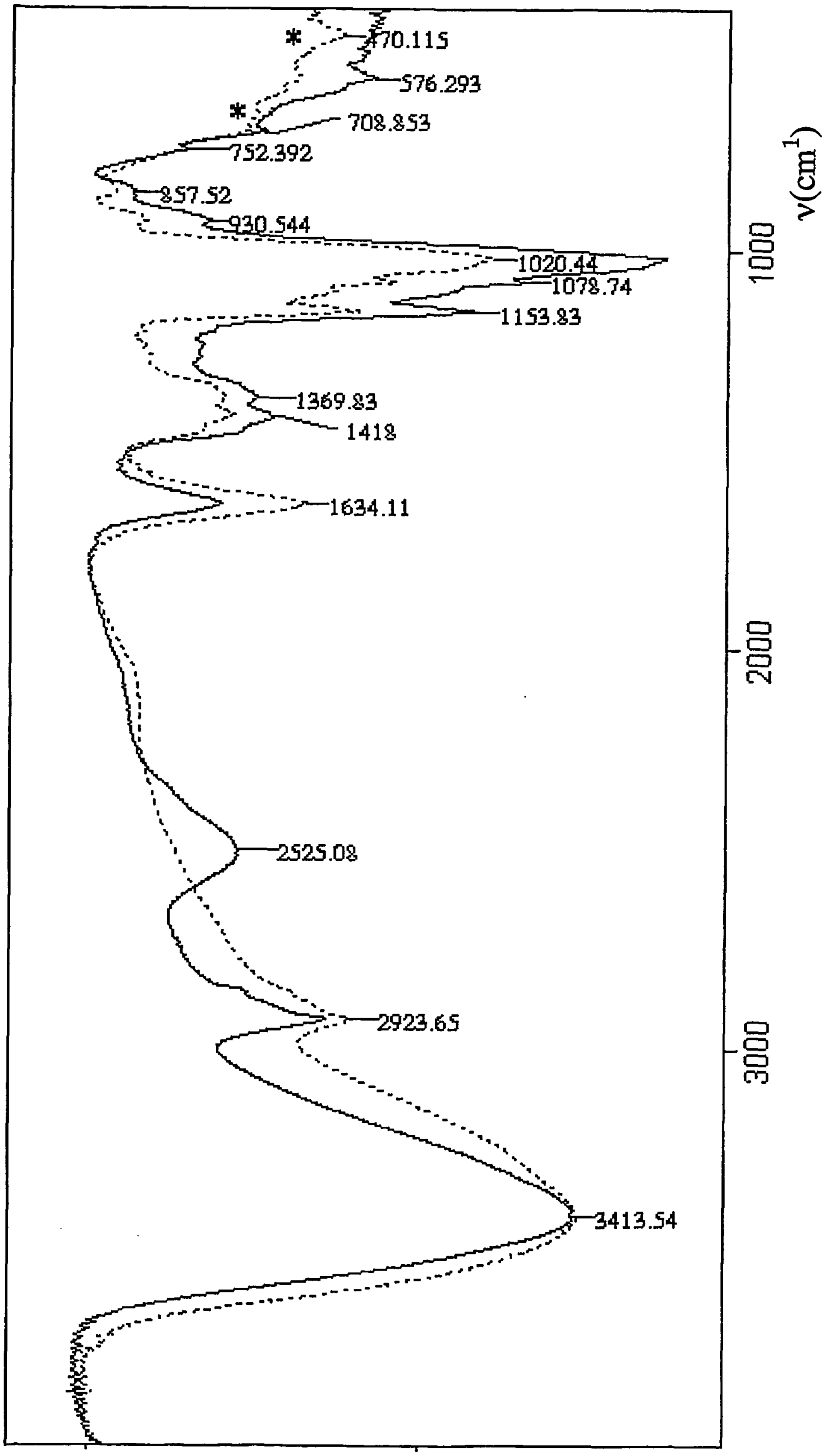


Figure 4: FTIR spectra: iron(III) complex with hydrogenated low-molar pullulan (a…) and its partially deuterated analogue (b___)

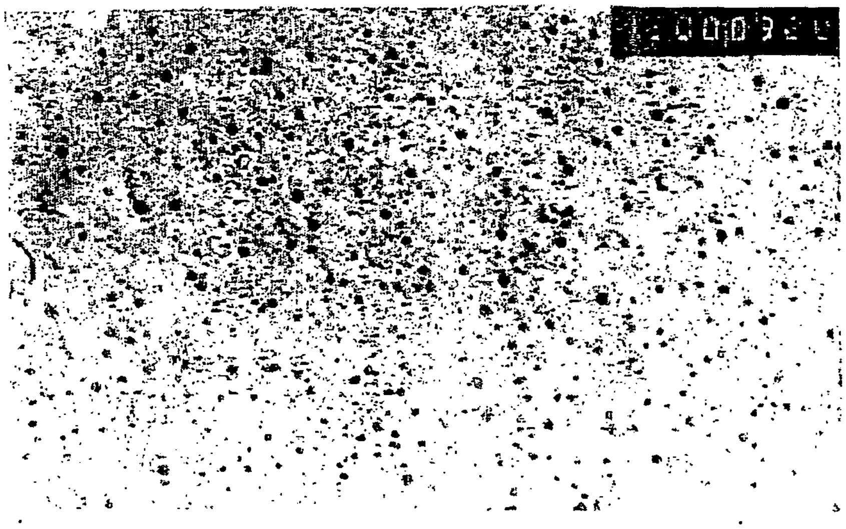

a)

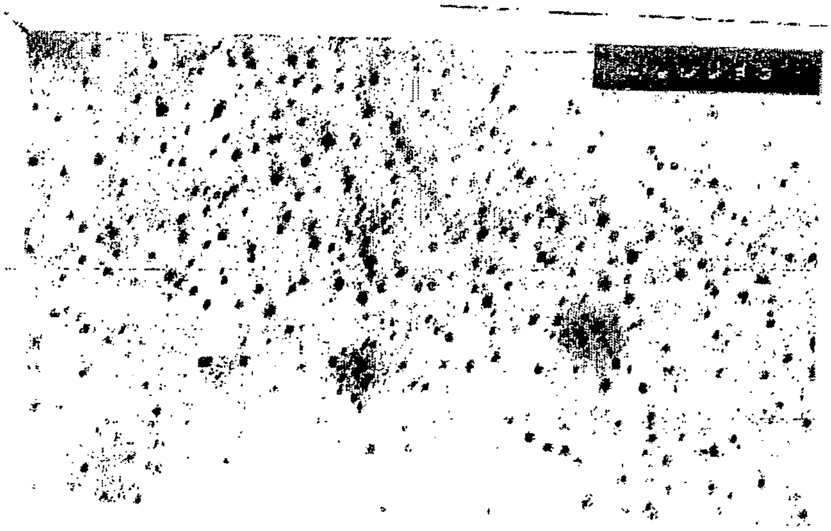

b)

Figure 5: Transmissional electronic micrograph (TEM) of
a. iron(III)- hydroxyde,
b. polynuclear complex of iron(III)- hydroxyde with pullulan oligomers

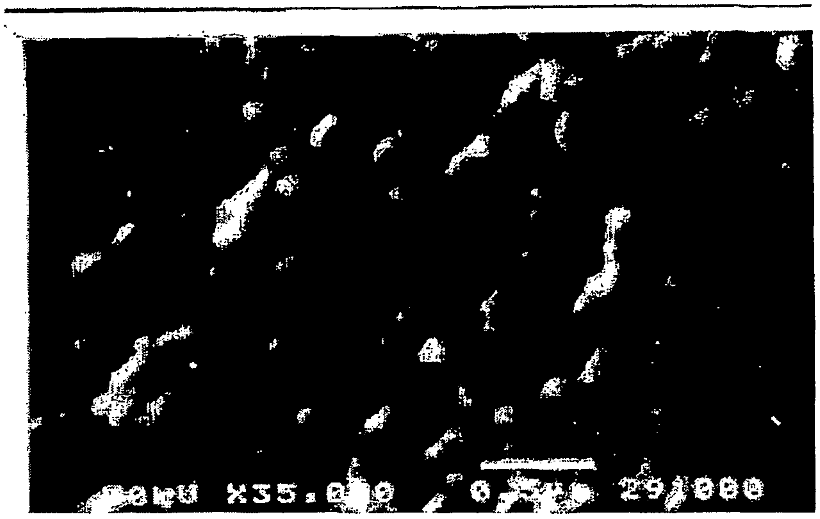

a)

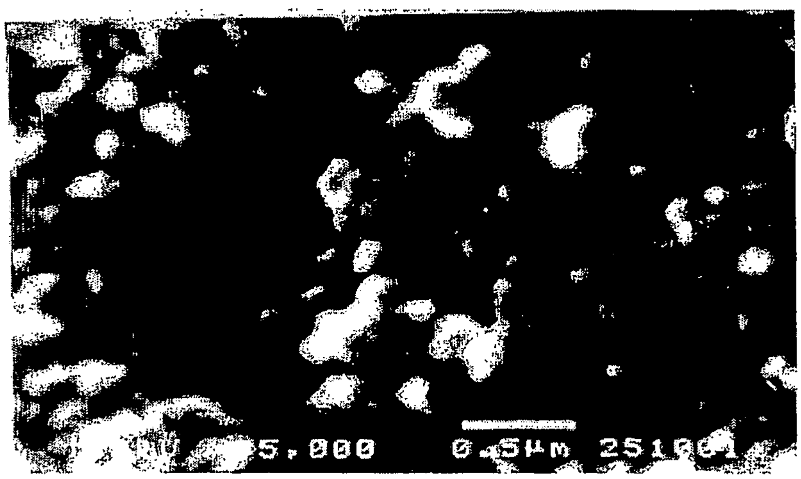

b)

Figure 6: Scanning electronic micrograph (SEM) of:

a. iron(III) -hydroxyde,
b. polynuclear complex of iron(III)- hydroxyde with pullulan oligomers